(19) Europäisches Patentamt
European Patent Office
Office européen des brevets

(11) **EP 3 618 801 B1**

(12) **EUROPEAN PATENT SPECIFICATION**

(45) Date of publication and mention
of the grant of the patent:
**15.03.2023  Bulletin 2023/11**

(21) Application number: **18742485.8**

(22) Date of filing: **17.07.2018**

(51) International Patent Classification (IPC):
**A61Q 11/00** (2006.01)    **A61K 8/49** (2006.01)
**A61K 8/81** (2006.01)    **A61K 8/25** (2006.01)
**A61K 8/19** (2006.01)

(52) Cooperative Patent Classification (CPC):
**A61K 8/494; A61K 8/19; A61K 8/25; A61K 8/8164;
A61Q 11/00;** A61K 2800/58

(86) International application number:
**PCT/EP2018/069408**

(87) International publication number:
**WO 2019/025180 (07.02.2019 Gazette 2019/06)**

(54) **TOOTHPASTE COMPOSITION COMPRISING PIGMENTS**

ZAHNPASTAZUSAMMENSETZUNG ENTHALTEND PIGMENTEN

COMPOSITION DE DENTIFRICE COMPRENANT DES PIGMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.08.2017  EP 17184738**

(43) Date of publication of application:
**11.03.2020  Bulletin 2020/11**

(73) Proprietors:
• **Unilever IP Holdings B.V.
3013 AL Rotterdam (NL)**
Designated Contracting States:
**AL AT BE BG CH CZ DK EE ES FI FR GR HR HU
IS LI LT LU LV MC MK NL NO PL PT RO RS SE SI
SK SM TR**
• **Unilever Global IP Limited
Wirral, Merseyside CH62 4ZD (GB)**
Designated Contracting States:
**CY DE GB IE IT MT**

(72) Inventor: **BARILI, Matteo
26841 Casalpusterlengo Lodi (IT)**

(74) Representative: **Tansley, Sally Elizabeth
Unilever Patent Group
Bronland 14
6708 WH Wageningen (NL)**

(56) References cited:
**EP-A1- 1 935 395      EP-A1- 2 380 565
WO-A1-2006/081926      US-A- 4 456 585**

• **DATABASE GNPD [Online] MINTEL; October
2012 (2012-10), Anonymous: "Advanced Fresh
Anticavity Toothpaste Gel", XP002776500,
Database accession no. 1910072**
• **DATABASE GNPD [Online] MINTEL; December
2005 (2005-12), Anonymous: "Gel Toothpaste",
XP002776501, Database accession no. 418649**

**Description**

The present invention relates to a tooth whitening composition.

Toothpaste compositions that whiten the teeth are available commercially. There are two principle modes of action for these toothpastes either by bleaching the teeth or by adding a colourant.

Oral care composition comprising blue pigments and soluble deposition aid for the pigment are disclosed in EP1935395, however, there remains the need for toothpastes that effectively whiten the teeth.

The present invention is concerned with improved compositions for cosmetic tooth whitening.

**Description of the Invention**

The present invention relates to an oral care composition comprising a polymeric deposition aid, a blue pigment and a green pigment in which the weight ratio of green pigment to blue pigment is 1:2 or greater, in which the total level of pigment is from 0.05 to 0.2 wt% of the total composition and in which the blue pigment is Phthalocyanine Blue and the green pigment is Phthalocyanine Green.

The invention further relates to the use of a blue pigment and a green pigment, at a wt% of green pigment to blue pigment of 1:2 or greater, to enhance the white appearance of teeth, wherein the total level of pigment is from 0.05 to 0.2 wt% of the total composition and wherein the blue pigment is Phthalocyanine Blue and the green pigment is Phthalocyanine Green.

**Detailed Description of the Invention**

Compositions according to the invention comprises a green and a blue pigment, wherein the blue pigment is Phthalocyanine Blue and the green pigment is Phthalocyanine Green. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

The blue pigment is Phthalocyanine Blue Pigment, Cl No. 74160, blue covarine.

The Green pigment is Phthalocyanine Green, preferably Phthalocyanine Green CI-74260.

Preferably the composition is a multiphase toothpaste in which the composition is free of dye.

The composition comprises a combination of green and blue pigment, the weight ratio of green pigment to blue pigment is 1:2 or greater, preferably greater than 2:3 most preferably the weight ratio of green pigment to blue pigment is from 2:3 to 3:2.

Compositions according to the invention comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:

Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions of the invention are preferably toothpastes.

Compositions according to the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

Preferably the composition, particularly a toothpaste, comprises a silica based abrasive. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 $m^2$/g and an oil absorption of about 70 - 150 $cm^3$/100 g, but

abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the composition generally ranges from 5-60% by weight, usually 5-20% by weight.

The composition, particularly if a toothpaste preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone. In the context of the present invention silica is particularly preferred.

The toothpaste composition will comprise further ingredients which are common in the art, such as:

antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.; anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants; Humectants such as glycerol, sorbitol, propyleneglycol, xylitol, lactitol etc.;
binders and thickeners such as sodium carboxymethyl-cellulose, hydroxyethyl cellulose (Natrosol®), xanthan gum, gum arabic etc. as well as synthetic polymers such as polyacrylates and carboxyvinyl polymers such as Carbopol®; polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

In a preferred embodiment of the invention the toothpaste composition is a multiphase composition comprising an inner coloured phase disposed within a visually clear outer phase.

Preferably the inner phase comprising carrier fluid, silica based thickener, a silica based abrasive and a pigment and the outer phase comprising carrier fluid, silica based thickener, a silica based abrasive.

In a preferred multiphase embodiment the inner phase constitutes from 3 to 20% by volume more preferably from 4 to 15%.

In a preferred embodiment the first composition is visually-clear. By this is meant that the inner, second composition can be seen through the outer, first composition.

The compositions of the toothpaste according to the invention are manufactured using standard processes. They may be extruded into a container for dispensing by equipment such as that discussed in detail in WO 99/01342, i.e. a coaxial nozzle assembly attached to standard equipment.

Preferably the composition of the invention have viscosities as measured on a Brookfield RV DV-1 viscometer fitted with a Helipath stand at 25°C and 5rpm using a spindle are from 150 000 Pa.s and 250 000 mPa.s. Such viscosities provide the best performance with regard to extrusion into the container and also from the container by the consumer.

The phases within this viscosity range are much more stable, physically during extrusion, that other phases.

If a multiphase product it is preferred if the first and second phases are similar in principle components, i.e. thickeners, actives, structurants and abrasives, however the levels of minors such as flavour may differ. The levels of principle components within each phase may also differ. Preferably the inner phase comprises carrier fluid, silica based thickener, silica based abrasive and pigment and the outer phase is similar to the inner phase but does not contain pigment.

An embodiment of the invention is now discussed in the non-limiting example.

Examples of the invention are illustrated by a number, comparative examples by a letter.

## EXAMPLES

The following Examples were prepared:

| Ingredient | level wt % Example A | level wt% Example 1 |
|---|---|---|
| Sorbitol 70% non-crystallizing grade | 65.00 | 65.00 |
| Hydrated Silica Medium Abrasivity | 8.00 | 8.00 |
| Hydrated Silica Thickening Med Viscosity | 6.00 | 6.00 |
| Hydrated Silica High Abrasivity | 1.65 | 1.65 |
| Peg-32 (Flakes) | 1.50 | 1.50 |
| PAS | 1.80 | 1.80 |
| Tri sodium Phosphate-12-hydrate | 1.10 | 1.10 |
| Methyl Vinyl Ether - Maleic anhydride Copolymers | 0.10 | 0.10 |
| Cellulose Gum SCMC 9H | 0.60 | 0.60 |
| Blue pigmentCl 74160 | 0.0625% | 0.0625% |
| Green pigmentCl 74260 dispersion | 0.017% | 0.070 % |
| Water and minors | **To 100.00000%** | **To 100.00000%** |

The level of whitening was measured by pre-cleaning teeth with a silica paste. The teeth were the dried and the L*, a,*b* measured using a chromameter. Teeth were then brushed with the Example toothpaste for 1 minute, rinsed and dried The L*, a,*b* was once again measured using a chromameter

$$\Delta E = \sqrt{(\Delta L^{*2} + \Delta a^{*2} + \Delta b^{*2})}$$

| Example | $\Delta E$ |
|---|---|
| A | 1.35 |
| 1 | 1.82 |

Figures are significantly different to P 0.05

Increased whitening of the teeth is achieved with the Example according to the invention.

## Claims

1. An oral care composition comprising a polymeric deposition aid, a blue pigment and a green pigment in which the wt % of green pigment to blue pigment is 1:2 or greater, in which the total level of pigment is from 0.05 to 0.2 wt% of the total composition and in which the blue pigment is Phthalocyanine Blue and the green pigment is Phthalocyanine Green.

**2.** An oral care composition according to claim 1 in which the weight ratio of blue pigment to green pigment is 1:1 or greater.

**3.** An oral care composition according to any preceding claim in which the composition is free of dye.

**4.** An oral care composition according to any preceding claim that is a toothpaste.

**5.** A toothpaste composition according to any preceding claim that is a multiphase composition comprising an inner coloured phase disposed within a visually clear outer phase.

**6.** A toothpaste composition according to any preceding claim that comprises a silica based abrasive.

**7.** A toothpaste composition according to claim 5 or claim 6 in which

 i) the inner phase comprising carrier fluid, silica based thickener, a silica based abrasive and a pigment,
 ii) the outer phase comprising carrier fluid, silica based thickener, a silica based abrasive.

**8.** Use of a blue pigment and a green pigment, at a wt% of green pigment to blue pigment of 1:2 or greater, to enhance the white appearance of teeth wherein the total level of pigment is from 0.05 to 0.2 wt% of the total composition and wherein the blue pigment is Phthalocyanine Blue and the green pigment is Phthalocyanine Green.


**Patentansprüche**

**1.** Mundpflegezusammensetzung, umfassend ein polymeres Abscheidungshilfsmittel, ein blaues Pigment und ein grünes Pigment, worin der Gewichtsprozentsatz von grünem Pigment zu blauem Pigment 1:2 oder größer ist, worin der gesamte Anteil des Pigments 0,05 bis 0,2 Gew.-% der Gesamtzusammensetzung beträgt und worin das blaue Pigment Phthalocyaninblau und das grüne Pigment Phthalocyaningrün ist.

**2.** Mundpflegezusammensetzung nach Anspruch 1, in welcher das Gewichtsverhältnis von blauem Pigment zu grünem Pigment 1:1 oder größer ist.

**3.** Mundpflegezusammensetzung nach irgendeinem vorhergehenden Anspruch, worin die Zusammensetzung frei von Farbstoff ist.

**4.** Mundpflegezusammensetzung nach irgendeinem vorhergehenden Anspruch, **dadurch gekennzeichnet, dass** sie eine Zahnpasta ist.

**5.** Zahnpastazusammensetzung nach irgendeinem vorhergehenden Anspruch, die eine mehrphasige Zusammensetzung darstellt, umfassend eine gefärbte innere Phase, die in einer visuell klaren äußeren Phase angeordnet ist.

**6.** Zahnpastazusammensetzung nach irgendeinem vorhergehenden Anspruch, die ein auf Siliziumdioxid basierendes Schleifmittel umfasst.

**7.** Zahnpastazusammensetzung nach Anspruch 5 oder Anspruch 6, worin

 i) die innere Phase eine Trägerflüssigkeit, ein auf Siliziumdioxid basierendes Verdickungsmittel, ein auf Siliziumdioxid basierendes Schleifmittel und ein Pigment umfasst,
 ii) die äußere Phase eine Trägerflüssigkeit, ein auf Siliziumdioxid basierendes Verdickungsmittel, ein auf Siliziumdioxid basierendes Schleifmittel umfasst.

**8.** Verwendung eines blauen Pigments und eines grünen Pigments bei einem Gewichtsprozentsatz von grünem Pigment zu blauem Pigment von 1:2 oder grö-ßer, um das weiße Erscheinungsbild der Zähne zu verbessern, wobei der gesamte Anteil des Pigments 0,05 bis 0,2 Gew.-% der Gesamtzusammensetzung beträgt und wobei das blaue Pigment Phthalocyaninblau und das grüne Pigment Phthalocyaningrün ist.

**Revendications**

1. Composition pour le soin oral comprenant une aide au dépôt polymère, un pigment bleu et un pigment vert dans laquelle le % en masse de pigment vert à pigment bleu est de 1:2 ou supérieur, dans laquelle la teneur totale en pigment est de 0,05 à 0,2 % en masse de la composition totale et dans laquelle le pigment bleu est le bleu de phtalocyanine et le pigment vert est le vert de phtalocyanine.

2. Composition pour le soin oral selon la revendication 1 dans laquelle le rapport en masse de pigment bleu à pigment vert est de 1:1 ou supérieur.

3. Composition pour le soin oral selon l'une quelconque des revendications précédentes, dans laquelle la composition est exempte de colorant.

4. Composition pour le soin oral selon l'une quelconque des revendications précédentes qui est un dentifrice.

5. Composition de dentifrice selon l'une quelconque des revendications précédentes qui est une composition à phases multiples comprenant une phase colorée interne disposée à l'intérieur d'une phase externe visuellement transparente.

6. Composition de dentifrice selon l'une quelconque des revendications précédentes qui comprend un abrasif à base de silice.

7. Composition de dentifrice selon la revendication 5 ou revendication 6 dans laquelle

   i) la phase interne comprend un fluide porteur, un épaississant à base de silice, un abrasif à base de silice et un pigment,
   ii) la phase externe comprend un fluide porteur, un épaississant à base de silice, un abrasif à base de silice.

8. Utilisation d'un pigment bleu et d'un pigment vert, à un % en masse de pigment vert à pigment bleu de 1:2 ou supérieur, pour améliorer l'apparence blanche de dents dans laquelle la teneur totale en pigment est de 0,05 à 0,2 % en masse de la composition totale et dans laquelle le pigment bleu est le bleu de phtalocyanine et le pigment vert est le vert de phtalocyanine.

**EP 3 618 801 B1**

**REFERENCES CITED IN THE DESCRIPTION**

**Patent documents cited in the description**

- EP 1935395 A
- WO 9901342 A